# EUROPEAN PATENT SPECIFICATION

(11) **EP 2 640 274 B1**
(45) Date of publication and mention of the grant of the patent: **30.03.2016**
(21) Application number: 11799317.0
(22) Date of filing: 15.11.2011
(51) Int. Cl.: A61B 18/18, B06B 1/06, G01S 15/89, A61B 18/00, A61B 8/08, A61B 8/12, G01S 7/52, A61B 18/14

(54) **INTERFERENCE REDUCTION AND SIGNAL TO NOISE RATIO IMPROVEMENT FOR ULTRASOUND CARDIAC ABLATION MONITORING**
INTERFERENZMINIMIERUNG UND VERBESSERTES SIGNAL-RAUSCH-VERHÄLTNIS FÜR ULTRASCHALL-HERZABLATIONSÜBERWACHUNG
RÉDUCTION DE BROUILLAGE ET AMÉLIORATION DU RAPPORT SIGNAL-BRUIT POUR UNE SURVEILLANCE D'ABLATION CARDIAQUE PAR ULTRASONS

(30) Priority: 18.11.2010 EP 10191687; 18.03.2011 EP 11158848
(43) Date of publication of application: 25.09.2013
(73) Proprietor: Koninklijke Philips N.V., 5656 AE Eindhoven (NL)
(72) Inventor: BUDZELAAR, Franciscus, Paulus, Maria, 5656 AE Eindhoven (NL); MIHAJLOVIC, Nenad, 5656 AE Eindhoven (NL); FOKKENROOD, Steven, Antonie, Willem, 5656 AE Eindhoven (NL)
(74) Representative: Steffen, Thomas
(86) International application number: PCT/IB2011/055084
(87) International publication number: WO 2012/066472

(56) References cited:
- US-A- 5 183 048
- US-A- 5 788 636
- US-A1- 2005 261 571
- US-A1- 2006 264 748
- US-A1- 2009 018 442
- US-B1- 6 425 867
- US-B1- 6 633 658

## Description

### FIELD OF THE INVENTION

The present invention relates to an, apparatus, method and computer program product for interference reduction in ultrasound cardiac ablation applications, especially for interference reduction during RF ablation using RF catheters having ultrasound transducers for monitoring the progress of lesions made to cardiac tissue.

### BACKGROUND OF THE INVENTION

Cardiac ablation technology as a common procedure for treating atrial fibrillation usually is based on an ablation device with an ablation electrode provided within a radiofrequency (RF) catheter for navigating within a patient's body. The ablation electrode is provided at the distal end of the catheter so that tissue located between the ablation electrode and an indifferent electrode positioned next to the patient's body can be treated. Combined with an imaging system, usually based on ultrasound (US), such an ablation device is aimed to provide lesions of a specific depth to the atrial wall of a patient's heart. The lesions formed by an ablation conduct much less than healthy tissue, and thus effectively break any electrical paths over which the signals that cause the fibrillation are conducted. Generally, the lesions that are made should penetrate the complete atrial wall resp. heart wall for this procedure to be an effective treatment for atrial fibrillation, wherein e.g. in humans, the atrial wall can be up to 8mm thick. However, a lesion that is made too deep can be lethal; e.g. the oesophagus is a critical organ that should not be affected. Therefore, an ultrasound (US) transducer coupled with the ablation device is provided, especially built into the ablation catheter, and, where applicable, integrated adjacent to the ablation electrode, in order to generate information related to the progress of the ablation treatment. That is to say, US monitoring can give the surgeon a feedback mechanism on the progress of a lesion, which may increase the success rate of the procedure. Nonetheless, RF ablation causes interferences with US signals, so that in many cases, US monitoring is not reliable or trustworthy enough, and tissue ablation resp. treatment of atrial fibrillation cannot be done effectively.

In other words, currently, in spite of any imaging system, these ablation procedures are performed without a proper mechanism to assess the exact progress of the lesion, as there is e.g. capacitive coupling of RF signals into US signals, i.e. RF signals interfere with US signals. This causes the surgeon to be very cautious, e.g. due to the danger of injury from overheating. Further, in case of underheating, the treatment is ineffective. Therefore, even if US monitoring is integrated in the ablation system, there remain a significant number of treatments which are not effective. In all these cases, the lesions could not have been made such that the electrical paths over which the signals that cause the fibrillation are conducted are effectively disrupted.

Therefore, a requirement for radiofrequency (RF) catheters is more adequate control of the lesion development in the tissue, especially in real-time during RF ablation. A system that can provide a real-time feedback of the lesion development as well as real-time information about the depth of the lesion, especially with respect to the thickness of the tissue at the treatment site, would prevent injury and death, e.g. also from overheating in RF catheter ablation procedures. As mentioned above, high-frequency ultrasound (US) can be used to monitor the progression of the lesion boundary in motion-mode (M-mode) imaging, but the referred disadvantages are not overcome yet. The RF signal interferes with the US signal such that tissue reflections cannot be seen easily.

Apparatuses for the reduction of noise and electromagnetic interference in ultrasonic images are known in the art.

US2005/0261571 discloses a combined RF source and ultrasound imaging apparatus wherein the RF source and the imaging device are gated, ie. if ultrasound is active then no RF energy is applied and vice versa;

US2009/018442 is related to phase locking the (RF noise generating) switching operations of power supplies to the transmit/receive events of an ultrasound image. Image processing may be applied for removing the locked-in noise in the imaging signal.

According to US-B1-6 633 658, image portions distorted by noise or interference are replaced by stored clear image frames or moved to the periphery of the image.

### SUMMARY OF THE INVENTION

It is an object of the present invention to provide an apparatus and a method for treating tissue based on RF ablation and for monitoring treatment progress and tissue characteristics based on ultrasound which enables a surgeon to provide lesions of adequate depth to the tissue. It is a further object of the present invention to reduce the danger of injury from overheating. It is another object of the present invention to reduce the effect of capacitive coupling of RF signals into US signals, especially in order to enhance ultrasound based monitoring of ablation depth. Also, it is an object of the present invention to provide an ultrasound cardiac ablation monitor which is less susceptible resp. prone to any interference between RF and US signals, and to facilitate US monitoring of tissue characteristics in general, also in context with treatment of any other tissue than atrial walls. In other words, it is an aim of the present invention to improve US monitoring when US signals interfere with any other signals, e.g. RF ablation signals.

At least one of these objects is achieved by an apparatus as claimed in claim 1, and a method for interference reduction as claimed in claim 4.

Thereby, the present invention is applicable, inter alia, for therapy concepts where ultrasound is used for monitoring e.g. tissue characteristics, in particular when there is a highly repetitive interference signal, so for instance an interference signal of a RF ablation device. In particular, in context with RF ablation, the problem solved by the present invention relies, inter alia, in the following. Usually, the RF signal interferes with the US signal such that tissue reflections cannot be seen easily, since the RF signal is of much larger amplitude compared to US tissue reflections. More specifically, the frequency of the RF ablation signal is about 450 kHz, and US lesion monitoring is performed with frequencies higher than 10 MHz. However, the RF signals contain high frequency harmonics which significantly affect the US signals in the bandwidth of the US transducer. Until now, it has not been possible to filter out the RF ablation signal coupled into the US signal with an analogue filter.

The invention is based, inter alia, on the following recognitions. The ablation signal and therefore the interference picked up by the ultrasound transducer are of a repetitive nature. Although the exact shape of the interference signal cannot be estimated on forehand, this shape changes only slowly in time. The main cause for changes of this interference signal is the change of impedance of the tissue due to lesion formation, and the changes in the tissue occur only slowly. In one illustrative example, considering an ultrasound system operating at 20 MHz in water offering a resolution of roughly 30 µm, the fastest motion in the tissue is caused by blood flowing through capillaries, which is less than 4.5 mm/s. This means that in case two echo scans are taken less than 3 ms apart, the loss of details caused by motion is negligible, as the extend of motion is in the order of 0,0135 mm, i.e. below the resolution of 30 µm. At frequencies of 10 MHz and higher, the typical penetration depths in tissue is limited to less than 1 cm. With a speed of sound of approximately 1500 m/s in tissue, this results in a typical measurement time of less than 13 µs. Therefore, in this illustrative example, the maximum number of echo scans that can be taken during a period of 3 ms and that will be almost identical is 230, resulting from the period of maximum 3 ms and the measurement time of less than 13 µs. Thus, several ultrasound scans can be performed, each delivering an at least approximately equal signal sequence, and these signals can be compared to any interference signals in order to obtain an averaged US echo signal and/or to synchronize US scans to RF ablation signals, as further elucidated in context with embodiments of the invention. Thereby, a major advantage is that the apparatus, system and device for interference reduction can be used with existing commonly used ablation systems, especially without modifications, even if these systems generate substantial RF interference. That is to say, it is not necessary to alter existing systems.

Thereby, the present invention proposes a mechanism in which several ultrasound (US) scans can be performed, especially in a rapid succession within such a time period that loss of detail due to tissue or fluid motion is lesser than the resolution provided by the ultrasound system. This can be done in a burst like mode, especially by considering the polarity of subsequent pulses. I.e., the US scans of each burst can be timed, and the bursts themselves can be timed as well. Thereby, interference reduction can be simply achieved by providing the pulses in a rapid succession, so that motion of tissue or patient movement does not have a significant negative effect on the quality of US echo signals.

According to a first preferred embodiment, combining detected interference signals with a respective ultrasound echo signal for providing a combined echo signal and averaging at least two of the combined echo signals in order to obtain an averaged echo signal with high signal to noise ratio can lead to better US based monitoring, especially of the ablation depth. In other words, a combined echo signal corresponds to a signal received from the transducer comprising the signal wanted for imaging and the interference signal. Thereby, it came into notice that it can be sufficient to average the echo signals of a limited number of US scans. Averaging the scans resp. signals can result in a better signal to noise ratio (SNR) of an echo signal since the US component is at least approximately the same in the subsequent scans while the interference signal and noise may be different. Averaging can provide reduced interference and thus reconstructed US echo signals. That is to say, in the practical circumstances of an ablation intervention, having short measurement times per scan and a low speed of objects to be tracked, according to the invention, in one example of an application, up to 230 scans can be taken that are almost mutually identical. However, much fewer scans may be required. Based on averaging, interference reduction can be simply achieved by increasing the signal to noise ratio, so that ultrasound echo signals can be obtained with a higher quality.

According to a second preferred embodiment which can be combined with the above first preferred embodiment, the ultrasound device can be connected to the ablation device in order to enable synchronization of excitation pulses to RF ablation signals so that a respective interference signal of interference between echo signals and ablation signals has a predetermined phase. Thus, by synchronizing a respective ultrasound excitation pulse to the ablation signals, the interference will have a predetermined phase, especially with respect to the recorded echo signals, which enables e.g. the phase of US signals to be shifted on purpose in relation to the phase of ablation signals. Based on synchronization, interference reduction can be simply achieved by taking into account the phase of interference signals, so that on purpose, the phase of echo signals can be adjusted in relation to the phase of the ablation signals.

Thus, the present invention reduces the unavoidable interference caused by the harmonics of the strong RF ablation signals on the ablation electrode which are coupled with ultrasound signals received from an ultrasound transducer, wherein the US transducer can reside within this ablation electrode. Therefore, the present invention also provides the advantages of fewer restrictions in US transducer arrangement as well as less requirements to shielding. At the same time, it increases the signal to noise ratio (SNR) of the measured US echo signals and therefore penetration depth of US signals into the cardiac tissue.

According to a third preferred embodiment which can be combined with any one of the above first and second preferred embodiments, a pulse generating device for interference reduction in radiofrequency (RF) ablation applications using ultrasound based monitoring can be provided, wherein the pulse generating device is arranged for receiving an RF ablation signal, receiving a start burst signal for starting a first burst of at least two ultrasound scans including ultrasound signals, generating excitation pulses, synchronizing the excitation pulses to said RF ablation signals so that an interference signal of interference between echo signals and ablation signals has a predetermined phase, and the pulse generating device can further be arranged for providing timing information to a signal processing unit in order to time said excitation pulses and the start of a subsequent scan and/or the start of a second burst of scans in relation to ablation signals. In other words, US signals can be provided in a burst like mode with subsequent pulses having different polarity. In the received scans resp. signals, the US component can be reversed for each subsequent scan while the interference signal will be the same. By subtracting signals in subsequent scans from each other, the US component can be doubled, while the interference signal is cancelled out. A resulting signal can be obtained which is based on an averaging of combined signals. The combined signals can be obtained from combining interference signals to US echo signals, wherein in the resulting signal, the ultrasound echo is amplified, especially doubled, and the interference is reduced, especially cancelled out.

According to a fourth preferred embodiment which can be combined with any one of the above first, second and third preferred embodiments, alternatively or additionally, US signals can be provided, especially in a burst mode, with subsequent pulses having the same polarity as the ultrasound echo signals but being slightly shifted in phase with respect to the signals of the ablation device. In the received scans, through synchronizing the ultrasound excitation pulses resp. the ultrasound signals, the US component remains at the same time (position) while the interference signal can be shifted. Again, by averaging the signals of a number of scans, the signal to noise ratio (SNR) of echo signals is improved.

According to a fifth preferred embodiment which can be combined with any one of the above first, second, third and fourth preferred embodiments, responsive to the polarity of excitation pulses, combined echo signals may have alternating positive and negative polarity, in order to add signals for which the positive excitation is used and to substract the ones with negative excitation, and/or combined echo signals have same polarity as the ultrasound echo signals according to excitation pulses each with same polarity. Thereby, interference reduction can be simply achieved by increasing the response signal and cancelling out interference. Additionally, the pulse generating device can be arranged to carry out synchronization of the excitation pulses to the RF ablation signals so that a respective interference signal of interference between echo signals and ablation signals has a predetermined phase, and the system can further be arranged to provide a start burst signal to said pulse generating device in order to trigger the pulse generating device to generate a sequence of synchronized ultrasound excitation pulses. Further, the phase of the combined echo signals can be shifted with respect to the ablation signal. Thereby, interference reduction can be simply achieved by conserving useful signals while decreasing noise level and level of interfered signals. The repetition rate can be chosen such that no echoes are recorded from previous excitation pulses.

Of course other interference reduction options can be used. For example, bursts can be carried out and repeated in specific time periods with specific scanning time, so that an appropriate synchronization method can be found for any application in which interference occurs.

The apparatus described above may be implemented as an apparatus including an ablation device, an US device, and several devices for averaging and/or synchronizing. As an alternative, any devices for averaging and/or synchronizing may be integrated in the ablations device and/or in the US device.

It shall be understood that the apparatus of claim 1, the method of claim 4, and the computer program of claim 12 have similar and/or identical preferred embodiments, in particular, as defined in the dependent claims.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows a schematic drawing of a conventional arrangement for cardiac ablation within a human body;
Fig. 2 shows a schematic drawing of the ablation system of Fig. 1 in conjunction with ultrasound monitoring;
Fig. 3 shows a schematic block diagram of a basic system setup for interference reduction according to the present invention;
Fig. 4 shows a schematic drawing of an arrangement for cardiac ablation according to the present invention;
Fig. 5 schematically shows examples of signals of a first embodiment of interference reduction technique;
Fig. 6 schematically shows a typical scan sequence of the first embodiment of interference reduction technique;
Fig. 7 schematically shows examples of signals of a second embodiment of interference reduction technique; and
Fig. 8 schematically shows a typical scan sequence of the second embodiment of interference reduction technique;

### DETAILED DESCRIPTION OF EMBODIMENTS

In the following embodiments, an enhanced interference reduction system is proposed, especially for tissue ablation applications where US imaging is influenced by RF signals so that a surgeon's treatment possibilities are restricted.

According to the embodiments, an averaging of combined signals is carried out and additionally, synchronization of ultrasound excitation pulses to ablation signals can be carried out. Hence, the interference reduction system is adapted to increase signal to noise ratio of US signals.

In the following, two embodiments using averaging as well as synchronizing are described, starting from a brief description of state of the art.

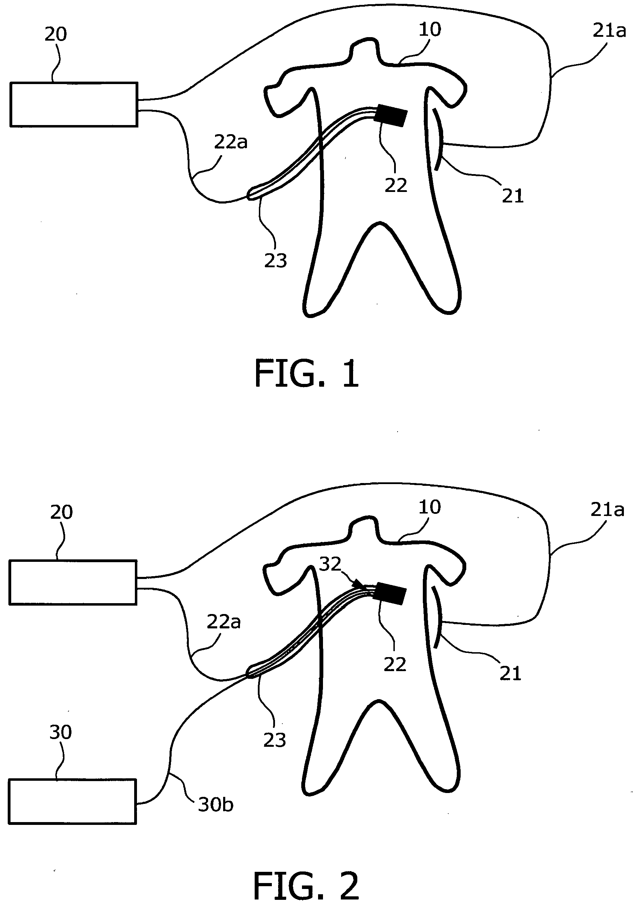

Fig. 1 shows a schematic drawing of a conventional arrangement for cardiac ablation within a human body 10, wherein an ablation electrode 22 is provided within a catheter 23 to be navigated within the human body 10 in order to treat tissue located between the ablation electrode 22 and an indifferent electrode 21. Ablation electrode 22 and indifferent electrode 21 are connected to an ablation device 20 via ablation wire 22a and connection 21a respectively.

Fig. 2 shows a schematic drawing of the ablation system of Fig. 1, but in conjunction with an ultrasound device 30. A small high frequency ultrasound transducer 32 is built into the ablation catheter 23 of an ablation device 20 in such a way that the use of that catheter 23 is not changed. Using this transducer 32, tissue, especially the heart wall, can be visualized during the ablation procedure. The ultrasound device 30 is physically connected to the ablation system 20 in such a way that the ultrasound transducer 32 is provided in direct proximity to an ablation electrode 22. Using an ultrasound device 30 in such close proximity of an ablation electrode 22 introduces a practical problem, as ablation is typically performed using a sinusoidal signal with a frequency between 440 and 480 KHz, and with a power of 20 to 50 watt. The tissue forms a load in the order of 100 to 300 ohm. The voltage required for ablation is therefore easily several tens of volts. But the harmonics of the base frequency are very difficult to suppress, and strong harmonics can be measured up to several megahertz. High frequency ultrasound in the range of 10 to 50 MHz is needed to visualize the heart wall with sufficiently resolution. Though the base frequency of the ablation is far outside the band of interest, the harmonics of it are within this band. In a practical system, it is thus extremely difficult, i.e. practically not feasible to sufficiently shield the US transducer 32 to reduce the interference of the ablation device 20 to a sufficiently low level. Consequently, there is a need for special mechanisms to reduce interference.

A second problem with such an ultrasound system is that the signal to noise ratio (SNR) limits the depths to which the ultrasound system can see resp. scan/analyze the tissue. Tissue attenuation increases with frequency, so a trade-off must be made between resolution, which means frequency, and penetration depth. Thus, with this technology of state of the art, due to interference and limited US visibility, a surgeon must be very cautions not to damage tissue or provide lesions that are too deep.

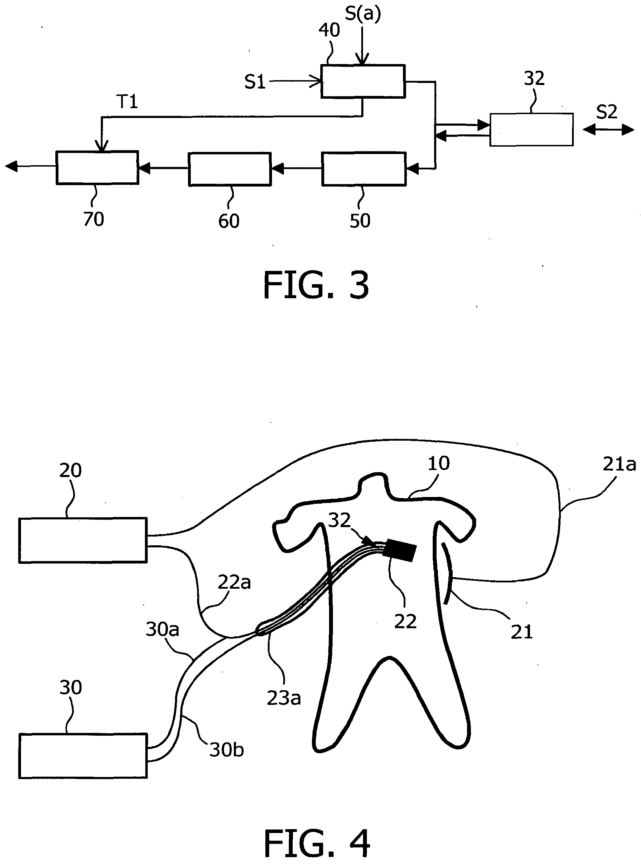

Fig. 3 shows a basic system setup according to the invention in order to reduce both problems mentioned above with the same approach. A start burst signal S1 triggers a pulser 40 to generate a sequence of pulses to excite the ultrasound transducer 32. The pulser 40 also receives an ablation signal S(a) so that it can synchronize the pulses to the ablation signal S(a). In other words, the ultrasound device is connected to or provided with a pulse generating device 40 which is arranged for receiving the RF ablation signals generated by an ablation device. The ultrasound transducer 32 is arranged for receiving an ultrasound echo signal generated in response to ultrasound excitation pulses, especially in response to each ultrasound excitation pulse. These acoustic signals provided by the transducer 32 can be forwarded to amplifier 50 in order to be processed as echo signals of which the signal to noise ratio is to be increased by averaging, wherein an interference signal of interferences between RF ablation signals and US signals is detected. Especially, the ultrasound transducer can be designed for detecting interference signals. Detection can be carried out with respect to each excitation pulse, or alternatively, with respect to specific excitation pulse, e.g. every other excitation pulse or every third excitation pulse. Detection can further be carried out with respect to a respective US echo signal. The ultrasound echo signals can be processed by the amplifier 50. The detected interference signals can be combined to respective ultrasound echo signals in order to obtain combined echo signals. In other words, a combined echo signal corresponds to a signal received from the transducer comprising the signal wanted for imaging and the interference signal. The combined echo signals can be amplified in the amplifier 50 and converted to digital signals in an A/D converter 60 in order to be provided to a signal processing unit 70. The signal processing unit 70 can be arranged for averaging at least two of said combined echo signals. That is to say, signal processing in signal processing unit 70 can take care of the required averaging and can also receive timing information T1 from the pulser 40 in order to time the start of the next scan resp. a subsequent burst. Thereby, the signal processing part can be performed in hardware or software. The implementation in hardware is preferred, as it can seriously reduce the data that needs to be transferred to the system using the ultrasound signal. All other modules are hardware modules.

With this technology, averaging as well as synchronization can be carried out. This means that the signal to noise ratio of US echo signals can be increased, and despite interference, an US transducer can be embedded in direct proximity of an ablation electrode.

Fig. 4 shows a regular ablation device 20 with a modified ablation catheter 23a, the modified ablation catheter 23a being arranged for providing both ablation electrode 22 and US transducer 32 in such a way that interference does not noticeably affect US imaging quality. The ablation electrode 22 and an US transducer 32 are provided at the distal end of the catheter 23a for navigating within a body in order to treat atrial fibrillation by providing lesions to cardiac tissue. The US transducer 32 can be embedded in the ablation electrode 22, and it is well shielded to minimize the interference that it picks up from the ablation signal. In other words, advantageously, the US transducer 32 can be used with existing commonly used ablation systems (as shown in Fig. 2), especially without the requirement of any modifications to the ablation electrode or catheter, even if these systems generate substantial RF interference. Thus, it is not necessary to substantially alter existing systems, so that the proposed apparatus, device and system for interference reduction basically can be implemented in all these commonly used ablations systems. The ultrasound device 30 resp. a pulser generates or causes generation of at least two scans resp. excitation pulses in a rapid succession, especially within such a time period that loss of detail due to tissue or fluid motion is lesser than the resolution provided by the ultrasound device. Thereby, the ultrasound device 30 can also be connected to a pulser arranged for generation of excitation pulses. These pulses can be synchronized to the ablation signal, wherein a connection 30a between the ultrasound device 30 and the ablation device 20 is provided, especially in the form of an additional cable from ablation wire 22a to the ultrasound device 30. Also, a pulser can be arranged for receiving the RF ablation signals.

Due to averaging, the signal to noise ratio (SNR) of echo signals can be increased. Noise is a truly random process, and adding *n* identical but noisy signals will increase the signal power by a factor of *n²*, but noise only with √*n*, therefore the signal to noise ratio can be increased with √*n*. So, by using e.g. two scans, SNR can be increased with 3 dB.

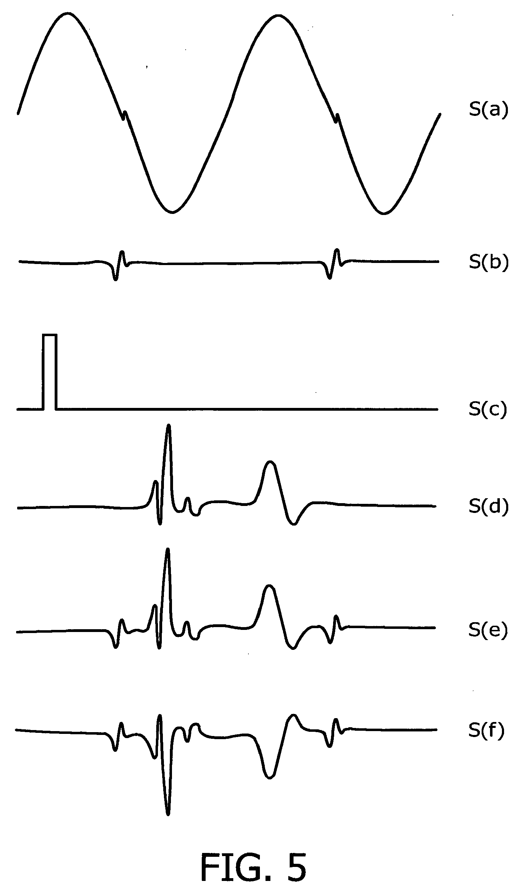

Fig. 5 schematically shows the sequence in which the signals according to a first embodiment of interference reduction technique can be provided. Several ultrasound scans are performed in a rapid succession (signal burst) with alternating polarity, wherein the scans can be performed by an ultrasound transducer communicating with a pulse generating device for generating excitation pulses, each scan being carried out in response to a respective excitation pulse. That is to say, responsive to positive and negative excitation pulses with alternating polarity, a combined echo signal S(e), S(f) can be provided with alternating positive and negative polarity, wherein a respective combined echo signal S(e), S(f) is composed of an ultrasound signal S(d) and an interference signal S(b). In the following, the principle of interference reduction technique according to the first embodiment is shortly explicated. A positive ultrasound excitation pulse S(c) is locked (i.e., synchronized) to the ablation signal S(a) so that the interference will have a fixed phase, especially with respect to the recorded US echo signals. Finally, the resulting echo signals S(e) for which the positive excitation is used are added, and the ones with negative excitation are subtracted.

Therein, Fig. 5 shows a very simplified example of the signals involved. Ablation signal S(a) with harmonics is typically several tens of volts. As example, cross-over distortion is shown at the negative edge of the sinusoidal signal, generating high level harmonics. Signal S(b) is the resulting interference signal picked up by the US transducer. This signal will typically be in the range of microvolts to millivolts. Signal S(c) shows a positive US excitation pulse for the transducer, especially locked to the ablation signal S(a). The US echo signal S(d) shows an example response when no interference would be present. Combined US echo signal S(e) shows the same signal, but with interference, so is the sum of signals S(d) and S(b), using a positive excitation pulse. Analogously, combined US echo signal S(f) is the result of a negative excitation signal. The resulting response is the same as signal S(e), but with opposite polarity, using a negative excitation pulse. The added interference however has the original polarity. Subtracting S(f) from S(e) results in a signal in which theoretically the response signal is doubled and the interference is cancelled out.

In practice, it might occur that some interference remains as the interference is not completely stationary and as there will be some jitter between the ultrasound excitation pulse and the ablation signal. Also, the response to a negative excitation pulse is not necessarily exactly the opposite to that invoked by a positive excitation pulse, e.g. because of non-linearities of tissue and/or transducer, or because of any imperfections in the electronic system.

Preferably, the repetition rate is chosen such that no echoes are recorded from previous excitation pulses. However, the total sequence of pulses should be as short as possible so that blood cell motion, which is assumed to be one of the fastest motions occurring in the visibility field of an US transducer, does not cause deterioration of the ultrasound image, or as the case may be, the US based monitoring.

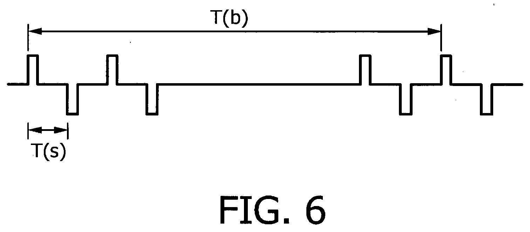

Fig. 6 shows an example of a typical scan sequence according to the first embodiment of interference reduction technique. In this example, each burst contains four scans, two positive scans and two negative scans. The burst repetition period T(b) can be in the order of e.g. 10 to 100 ms. More specifically, the burst repetition period T(b) can also be in the order of e.g. 1 ms or less, if high scan rates are advantageous in order to reduce interference, which might depend on the interference signal. The scan repetition period T(s), i.e. the time between two consecutive scans, can be in the order of e.g. 10 µs to 100 µs.

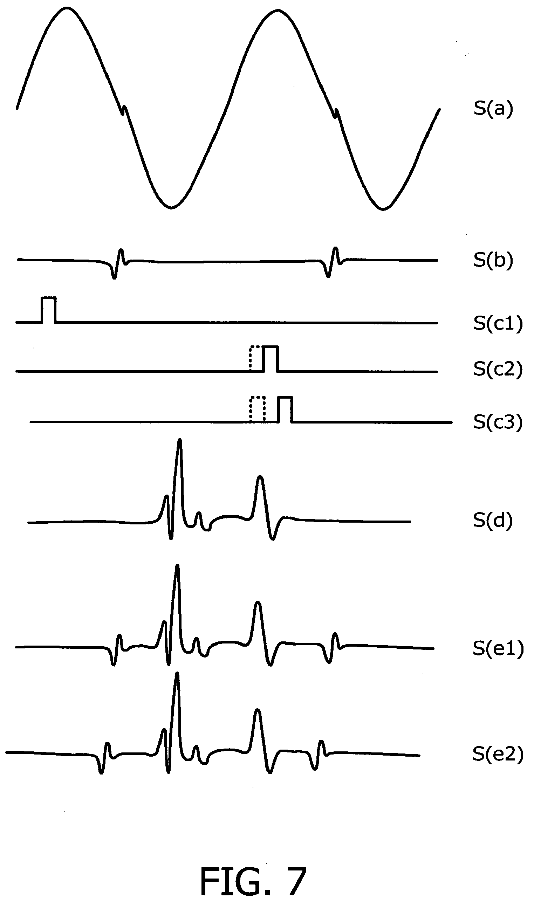

Fig. 7 schematically shows the sequence in which the signals according to a second embodiment of interference reduction technique can be provided. Several ultrasound scans are performed in a rapid succession (signal burst) with the same polarity as the ultrasound echo signals S(d). That is to say, responsive to excitation pulses each having same polarity, combined echo signals S(e1), S(e2) are provided with same polarity. In the following, the principle of interference reduction technique according to this second embodiment is shortly explicated. Likewise to the first embodiment, a respective ultrasound excitation pulse S(c1), S(c2), S(c3) is synchronized to the ablation signal, but for each pulse, the phase with respect to the ablation system is shifted on purpose. Therefore, the RF interference will also have a slightly shifted phase with respect to the recorded echo signals S(d). Finally, the resulting echo signals S(e1), S(e2) for which the positive excitation is used are averaged, and the interfered signal decreases if more averaging is done.

Therein, Fig. 7 shows a very simplified example of the signals involved. Signal S(a) is the ablation signal with harmonics. As example, cross-over distortion is shown at the negative edge of the sinusoidal signal, generating high level harmonics. Signal S(b) is the resulting interference signal picked up by the transducer. Signals S(c1), S(c2) and S(c3) show first, second and third excitation pulse respectively for the transducer. Signal S(d) shows an example for an US response (echo signal) when no interference would be present. Signal S(e1) shows the same signal, but with interference, obtained when first excitation pulse, especially according to signal S(c1), is applied. Signal S(e2) shows a signal with interference when second excitation pulse, especially according to signal S(c2), is applied. It can be seen that interfered signal S(e2) is a bit shifted with respect to interfered signal S(e1). The resulting response represents an average value of signals S(e1) and S(e2) and of other similarly generated signals. In such a way, a useful signal will remain, while noise level and level of an interfered signal will decrease as the number of averaging increases.

As mentioned in context with Fig. 5, the repetition rate can be chosen such that no echoes are recorded from previous excitation pulses. However, the total sequence of pulses should be as short as possible so that blood cell motion, which is assumed to be one of the fastest motions occurring in the visibility field of an US transducer, does not cause deterioration of the ultrasound image, or as the case may be, the US based monitoring.

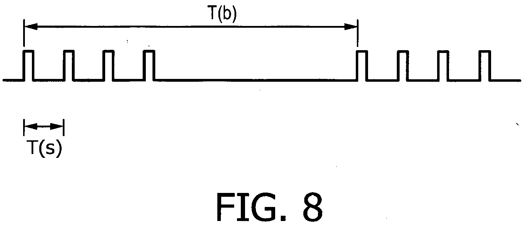

Fig. 8 shows an example of a typical scan sequence according to the first embodiment of interference reduction technique. In this example, each burst contains four positive scans, but the number of scans can be varied also. As in context with the first embodiment, the burst repetition period T(b) can be in the order of e.g. 10 to 100 ms. The scan repetition period T(s), i.e. the time between two consecutive scans, can be in the order of e.g. 10 µs to 100 µs.

It shall be understood that there is a sequence in which signals according to a third embodiment of interference reduction technique can be provided, the third embodiment being a combination of the first and second embodiments, i.e. positive and negative scans as well as shifting. In each pair of subsequent pulses, a positive and a negative excitation pulse is used, and the resulting echoes are subtracted. This suppresses the interference while increasing the SNR. However, some residual interference might be unavoidable. For the next pair, the phase with respect to the ablation system is slightly shifted, and the same procedure is repeated. The residual interference has the same strength but a different phase compared to the previous pair. Therefore, by averaging these pairs of pulses, the residual interference can be suppressed further than in the first embodiment.

In summary, in cardiac ablation for treatment of atrial fibrillation where lesions have to be made to the heart wall, an ultrasound monitoring mechanism is adapted to assess the progress of the lesion, so that a surgeon can provide lesions with adequate depth, wherein interference caused by an ablation device is reduced and signal to noise ratio of echo signals is improved. In other words, in RF applications where US imaging is used, an interference reduction system is adapted to at least substantially cancel out interference effects, so that US based monitoring is enhanced, especially monitoring of ablation depth.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single processor, sensing unit or other unit may fulfill the functions of several items recited in the claims. The mere fact that certain measures are recited in mutually different dependent claims does not indicate that a combination of these measures cannot be used to advantage.

It is noted that the proposed solution according to the above embodiments can be implemented at least partially in software modules at the relevant functional blocks of Fig. 3. The resulting computer program product may comprise code means for causing a computer to carry out the steps of the above procedures of functions of Fig. 3. Hence, the procedural steps are produced by the computer program product when run on the computer.

A computer program may be stored/distributed on a suitable medium, such as an optical storage medium or a solid-state medium supplied together with or as part of other hardware, but may also be distributed in other forms, such as via the Internet or other wired or wireless telecommunication systems.

In cardiac ablation for treatment of atrial fibrillation where lesions have to be made to the heart wall, an ultrasound monitoring mechanism is adapted to assess the progress of the lesion, so that a surgeon can provide lesions with adequate depth, wherein interference caused by an ablation device is reduced and signal to noise ratio of echo signals is improved.

## Claims

1. An apparatus for interference reduction in radio frequency (RF) ablation applications using real-time ultrasound based monitoring, said apparatus comprising:
- an ablation device (20) arranged for generating RF ablation signals (S(a)) supplied to an ablation electrode (22),
- an ultrasound device (30);
- an ultrasound transducer (32) connected to said ultrasound device (30);
wherein said apparatus is arranged for generating at least two ultrasound excitation pulses (S(c); S(c1), S(c2), S(c3)) in order to excite said ultrasound transducer (32), said ultrasound transducer (32) being arranged for performing an ultrasound scan for each ultrasound excitation pulse, each ultrasound scan including ultrasound signals (S2), and for receiving at least two combined ultrasound signals (S(e), S(e1), S(e2), S(f));
wherein the received combined ultrasound signals each include an interference signal (S(b)) of interference between said RF ablation signals (S(a)) and an ultrasound echo signal (S(d)) in response to an ultrasound excitation pulse,
wherein at least one received combined ultrasound signal is processed with at least another one received combined ultrasound signal in order to reduce the negative effect on ultrasound based monitoring that would be caused by said interference signal (S(b)).

2. The apparatus according to claim 1, wherein said apparatus is arranged for processing at least two of said combined ultrasound signals by averaging in order to obtain an averaged echo signal with high signal to noise ratio.

3. The apparatus according to claim 1, wherein said ultrasound device (30) is connected to said ablation device (20) in order to enable synchronization of said excitation pulses to said RF ablation signals so that a respective interference signal (S(b)) of interference between echo signals (S(d)) and ablation signals (S(a)) has a predetermined phase.

4. A method of reducing interference in radio frequency (RF) ablation applications using real-time ultrasound based monitoring, said method comprising:
a. generating RF ablation signals S(a) and detecting said RF ablation signals S(a);
b. generating at least two ultrasound excitation pulses (S(c); S(c1), S(c2), S(c3)), and providing said ultrasound excitation pulses to an ultrasound transducer (32) for performing ultrasound scans in response to said ultrasound excitation pulses;
c. receiving an at least two combined ultrasound signal (S(e), S(e1), S(e2), S(f)), wherein the received combined ultrasound signals each include an interference signal (S(b)) of interference between said RF ablation signals (S(a)) and an ultrasound echo signal (S(d)) in response to an ultrasound excitation pulse;
d. processing at least one received combined ultrasound signal with at least another one received combined ultrasound signal in order to reduce a negative effect on ultrasound based monitoring that would be caused by said interference signal (S(b)).

5. The method of claim 4,:
wherein the processing includes averaging at least two of said combined ultrasound signals to obtain an averaged echo signal with high signal to noise ratio.

6. The method according to claim 5, wherein one of the at least two combined ultrasound signals is responsive to a positive excitation pulse and another one of the at least two combined ultrasound signals is responsive to a negative excitation pulse, the positive excitation pulse and the negative excitation pulse having alternating polarity.

7. The method according to claim 6, wherein said averaging is carried out in such a way that said combined ultrasound signals for which said positive excitation is used are added, and the ones with negative excitation are subtracted, obtaining a resulting signal in which the ultrasound echo is amplified, and in which the interference is reduced.

8. The method according to claim 5, further comprising:
synchronizing said ultrasound excitation pulses to said ablation signal so that interference signal (S(b)) has a predetermined phase.

9. The method according to claim 5, wherein responsive to excitation pulses each having same polarity, combined ultrasound signals (S(e), S(f); S(e1), S(e2)) are provided with same polarity as said ultrasound echo signals (S(d)), and
wherein said averaging is carried out in such a way that said combined ultrasound signals for which the positive excitation is used are averaged, obtaining a resulting signal.

10. The method according to claim 5, the method further comprising:
synchronizing said ultrasound excitation pulses to said ablation signal, wherein the phase of said ultrasound echo signals (S(d)) is shifted with respect to said ablation signals so that said interference signal (S(b)) will have a shifted phase with respect to said ultrasound echo signals (S(d)).

11. The method according to claim 5, the method further comprising:
- before said step of averaging, amplifying said combined ultrasound signal;
- before said step of averaging, converting said combined ultrasound signal to a digital ultrasound signal;
- after said step of averaging, providing timing information (T1) from said pulse generating device (40) to said signal processing unit (70), and synchronizing said ultrasound excitation pulses to said ablation signal,
wherein generating at least two ultrasound excitation pulses (S(c); S(c1), S(c2), S(c3)) in a rapid succession is done in a burst like mode, each burst containing at least four scans, each scan being preferably less than 0,1 ms apart to a subsequent scan, and each burst being preferably more than 1 ms apart to a subsequent burst, said pulse generating device (40) receiving a start burst signal (S1) in order to generate a sequence of excitation pulses, said timing information (T1) being used for timing the start of a subsequent burst.

12. A computer program product comprising code means for producing the steps of claim 4 when run on a computing device as used in an apparatus according to claim 1.

## Patentansprüche

1. Gerät zur Interferenzminimierung bei Hochfrequenz- (HF-) Ablationsanwendungen unter Anwendung einer ultraschallbasierten Überwachung, wobei das genannte Gerät Folgendes umfasst:
- eine Ablationsvorrichtung (20), die dafür ausgelegt ist, HF-Ablationssignale (S(a)) zu erzeugen, die einer Ablationselektrode (22) zugeführt werden,
- eine Ultraschallvorrichtung (30);
- einen Ultraschallwandler (32), der mit der genannten Ultraschallvorrichtung (30) verbunden ist;
wobei das genannte Gerät dafür ausgelegt ist, mindestens zwei Ultraschallanregungsimpulse (S(c); S(c1), S(c2), S(c3)) zu erzeugen, um den genannten Ultraschallwandler (32) anzuregen, wobei der genannte Ultraschallwandler (32) dafür ausgelegt ist, einen Ultraschall-Scan für jeden Ultraschallanregungsimpuls durchzuführen, wobei jeder Ultraschall-Scan Ultraschallsignale (S2) umfasst, und mindestens zwei kombinierte Ultraschallsignale (S(e), S(e1), S(e2), S(f)) zu empfangen;
wobei die empfangenen kombinierten Ultraschallsignale jeweils ein Interferenzsignal (S(b)) der Interferenz zwischen den genannten HF-Ablationssignalen (S(a)) und einem Ultraschallechosignal (S(d)) in Reaktion auf einen Ultraschallanregungsimpuls umfassen;
wobei mindestens ein empfangenes kombiniertes Ultraschallsignal mit mindestens einem weiteren empfangenen kombinierten Ultraschallsignal verarbeitet wird, um die negative Auswirkung auf die ultraschallbasierte Überwachung zu minimieren, die durch das genannte Interferenzsignal (S(b)) verursacht würde.

2. Gerät nach Anspruch 1, wobei das genannte Gerät dafür ausgelegt ist, mindestens zwei der genannten kombinierten Ultraschallsignale durch Mittelwertbildung zu verarbeiten, um ein gemitteltes Echosignal mit hohem Signal-Rausch-Verhältnis zu erlangen.

3. Gerät nach Anspruch 1, wobei die genannte Ultraschallvorrichtung (30) mit der genannten Ablationsvorrichtung (20) verbunden ist, um eine Synchronisierung der genannten Anregungsimpulse mit den genannten HF-Ablationssignalen zu ermöglichen, so dass ein jeweiliges Interferenzsignal (S(b)) der Interferenz zwischen Echosignalen (S(d)) und Ablationssignalen (S(a)) eine vorgegebene Phase hat.

4. Verfahren zur Interferenzminimierung bei Hochfrequenz- (HF-) Ablationsanwendungen unter Anwendung einer ultraschallbasierten Überwachung, wobei das genannte Verfahren Folgendes umfasst:
a. Erzeugen von HF-Ablationssignalen S(a) und Detektieren der genannten HF-Ablationssignale S(a);
b. Erzeugen von mindestens zwei Ultraschallanregungsimpulsen (S(c); S(c1), S(c2), S(c3)) und Zuführen der genannten Ultraschallanregungsimpulse zu einem Ultraschallwandler (32) zum Durchführen von Ultraschall-Scans in Reaktion auf die genannten Ultraschallanregungsimpulse;
c. Empfangen von mindestens zwei kombinierten Ultraschallsignalen (S(e), S(e1), S(e2), S(f)), wobei die empfangenen kombinierten Ultraschallsignale jeweils ein Interferenzsignal (S(b)) der Interferenz zwischen den genannten HF-Ablationssignalen (S(a)) und einem Ultraschallechosignal (S(d)) in Reaktion auf einen Ultraschallanregungsimpuls umfassen;
d. Verarbeiten von mindestens einem empfangenen kombinierten Ultraschallsignal mit mindestens einem weiteren empfangenen kombinierten Ultraschallsignal, um die negative Auswirkung auf die ultraschallbasierte Überwachung zu minimieren, die durch das genannte Interferenzsignal (S(b)) verursacht würde.

5. Verfahren nach Anspruch 4,
wobei das Verarbeiten die Mittelwertbildung von mindestens zwei der genannten kombinierten Ultraschallsignale umfasst, um ein gemitteltes Echosignal mit hohem Signal-Rausch-Verhältnis zu erlangen.

6. Verfahren nach Anspruch 5, wobei eines der mindestens zwei kombinierten Ultraschallsignale auf einen positiven Anregungsimpuls reagiert und ein anderes der mindestens zwei kombinierten Ultraschallsignale auf einen negativen Anregungsimpuls reagiert, wobei der positive Anregungsimpuls und der negative Anregungsimpuls eine abwechselnde Polarität haben.

7. Verfahren nach Anspruch 6, wobei die genannte Mittelwertbildung auf derartige Weise durchgeführt wird, dass die genannten kombinierten Ultraschallsignale, für die die genannte positive Anregung verwendet wird, addiert werden, und diejenigen mit negativer Anregung subtrahiert werden, wodurch ein resultierendes Signal erlangt wird, in dem das Ultraschallecho verstärkt wird und in dem die Interferenz minimiert wird.

8. Verfahren nach Anspruch 5, das weiterhin Folgendes umfasst:
Synchronisieren der genannten Ultraschallanregungsimpulse mit dem genannten Ablationssignal, so dass das Interferenzsignal (S(b)) eine vorgegebene Phase hat.

9. Verfahren nach Anspruch 5, wobei in Reaktion darauf, dass die Anregungsimpulse jeweils die gleiche Polarität haben, kombinierte Ultraschallsignale (S(e), S(f); S(e1), S(e2)) mit der gleichen Polarität wie die genannten Ultraschallechosignale (S(d)) bereitgestellt werden, und
wobei die genannte Mittelwertbildung auf derartige Weise durchgeführt wird, dass die kombinierten Ultraschallsignale, für die die positive Anregung verwendet wird, gemittelt werden, wodurch ein resultierendes Signal erlangt wird.

10. Verfahren nach Anspruch 5, wobei das Verfahren weiterhin Folgendes umfasst:
Synchronisieren der genannten Ultraschallanregungsimpulse mit dem genannten Ablationssignal, wobei die Phase der genannten Ultraschallechosignale (S(d)) in Bezug auf die genannten Ablationssignale verschoben wird, so dass das genannte Interferenzsignal (S(b)) in Bezug auf die genannten Ultraschallechosignale (S(d)) eine verschobene Phase haben wird.

11. Verfahren nach Anspruch 5, wobei das Verfahren weiterhin Folgendes umfasst:
- vor dem genannten Schritt der Mittelwertbildung Verstärken des genannten kombinierten Ultraschallsignals;
- vor dem genannten Schritt der Mittelwertbildung Umwandeln des genannten kombinierten Ultraschallsignals in ein digitales Ultraschallsignal;
- nach dem genannten Schritt der Mittelwertbildung Bereitstellen von Zeitinformationen (T1) von der genannten Impulserzeugungsvorrichtung (40) für die genannte Signalverarbeitungseinheit (70), und Synchronisieren der genannten Ultraschallanregungsimpulse mit dem genannten Ablationssignal,
wobei das Erzeugen von mindestens zwei Ultraschallanregungsimpulsen (S(c); S(c1), S(c2), S(c3)) in rascher Folge in einem Burstmodus erfolgt, wobei jeder Burst mindestens vier Scans enthält, wobei jeder Scan vorzugsweise weniger als 0,1 ms von einem nachfolgenden Scan getrennt ist, und wobei jeder Burst vorzugsweise mehr als 1 ms von einem nachfolgenden Burst getrennt ist, wobei die genannte Impulserzeugungsvorrichtung (40) ein Startburstsignal (S1) empfängt, um eine Sequenz von Anregungsimpulsen zu erzeugen, wobei die genannten Zeitinformationen (T1) für die Zeitsteuerung des Beginns eines nachfolgenden Bursts verwendet werden.

12. Computerprogrammprodukt mit Codemitteln zum Erzeugen der Schritte nach Anspruch 4, wenn es auf einer Computervorrichtung ausgeführt wird, wie sie in einem Gerät nach Anspruch 1 verwendet wird.

## Revendications

1. Appareil de réduction de brouillage dans des applications d'ablation par radiofréquence (RF) utilisant une surveillance ultrasonore en temps réel, ledit appareil comprenant :
- un dispositif d'ablation (20) conçu pour générer des signaux d'ablation RF (S(a)) fournis à une électrode d'ablation (22),
- un dispositif ultrasonore (30) ;
- un transducteur ultrasonore (32) branché sur ledit dispositif ultrasonore (30) ;
dans lequel ledit appareil est conçu pour générer au moins deux impulsions d'excitation ultrasonores (S(c) ; S(c1), S(c2), S(c3)) afin d'exciter ledit transducteur ultrasonore (32), ledit transducteur ultrasonore (32) étant conçu pour effectuer un balayage ultrasonore pour chaque impulsion d'excitation ultrasonore, chaque balayage ultrasonore incluant des signaux ultrasonores (S2), et pour recevoir au moins deux signaux ultrasonores combinés (S(e), (S(e1), S(e2), S(f)) ;
dans lequel les signaux ultrasonores combinés reçus incluent chacun un signal (S(b)) de brouillage entre lesdits signaux d'ablation RF (S(a)) et un signal d'écho ultrasonore (S(d)) en réponse à une impulsion d'excitation ultrasonore,
dans lequel au moins un signal ultrasonore combiné reçu est traité à l'aide d'au moins un autre signal ultrasonore combiné reçu afin de réduire l'effet négatif sur la surveillance ultrasonore qui pourrait être provoqué par ledit signal de brouillage (S(b)).

2. Appareil selon la revendication 1, dans lequel ledit appareil est conçu pour traiter au moins deux desdits signaux ultrasonores combinés par moyennage afin d'obtenir un signal d'écho moyenné avec un rapport de signal sur bruit élevé.

3. Appareil selon la revendication 1, dans lequel ledit dispositif ultrasonore (30) est branché sur ledit dispositif d'ablation (20) afin de permettre la synchronisation desdites impulsions d'excitation avec lesdits signaux d'ablation RF de telle sorte qu'un signal de brouillage respectif (S(b)) de brouillage entre les signaux d'écho (S(d)) et les signaux d'ablation (S(a)) a une phase prédéterminée.

4. Procédé de réduction de brouillage dans des applications d'ablation par radiofréquence (RF) utilisant une surveillance ultrasonore en temps réel, ledit procédé comprenant :
a. la génération de signaux d'ablation RF (S(a) et la détection desdits signaux d'ablation RF S(a) ;
b. la génération d'au moins deux impulsions d'excitation ultrasonores (S(c) ; S(c1), S(c2), S(c3)), et la fourniture desdites impulsions d'excitation ultrasonores à un transducteur ultrasonore (32) pour effectuer un balayage ultrasonore en réponse auxdites impulsions d'excitation ultrasonores ;
c. la réception d'au moins deux signaux ultrasonores combinés (S(e), S(e1), S(e2), S(f)), dans laquelle les signaux ultrasonores combinés reçus incluent chacun un signal de brouillage (S(b)) de brouillage entre lesdits signaux d'ablation RF (S(a)) et un signal d'écho ultrasonore (S(d)) en réponse à une impulsion d'excitation ultrasonore ;
d. le traitement d'au moins un signal ultrasonore combiné reçu à l'aide d'au moins un autre signal ultrasonore combiné reçu afin de réduire un effet négatif sur la surveillance ultrasonore qui pourrait être provoqué par ledit signal de brouillage (S(b)).

5. Procédé selon la revendication 4 :
dans lequel le traitement inclut le moyennage d'au moins deux desdits signaux ultrasonores combinés afin d'obtenir un signal d'écho moyenné avec un rapport de signal sur bruit élevé.

6. Procédé selon la revendication 5, dans lequel l'un des au moins deux signaux ultrasonores combinés est sensible à une impulsion d'excitation positive et un autre des au moins deux signaux ultrasonores combinés est sensible à une impulsion d'excitation négative, l'impulsion d'excitation positive et l'impulsion d'excitation négative ayant une polarité alternée.

7. Procédé selon la revendication 6, dans lequel ledit moyennage est réalisé de telle manière que lesdits signaux ultrasonores combinés pour lesquels ladite excitation positive est utilisée sont additionnés, et ceux ayant une excitation négative sont soustraits, obtenant un signal résultant dans lequel l'écho ultrasonore est amplifié, et dans lequel le brouillage est réduit.

8. Procédé selon la revendication 5, comprenant en outre :
la synchronisation desdites impulsions d'excitation ultrasonores avec ledit signal d'ablation de telle sorte que le signal de brouillage (S(b)) a une phase prédéterminée.

9. Procédé selon la revendication 5, dans lequel en réponse aux impulsions d'excitation ayant chacune la même polarité, les signaux ultrasonores combinés (S(e), S(f) (S(e1), S(e2)) sont fournis avec la même polarité que lesdits signaux d'écho ultrasonores (S(d)), et
dans lequel ledit moyennage est réalisé de telle manière que lesdits signaux ultrasonores combinés pour lesquels l'excitation positive est utilisée sont moyennés, obtenant un signal résultant.

10. Procédé selon la revendication 5, le procédé comprenant en outre :
la synchronisation desdites impulsions d'excitation ultrasonores avec ledit signal d'ablation, dans lequel la phase desdits signaux d'écho ultrasonores (S(d)) est décalée par rapport auxdits signaux d'ablation de telle sorte que ledit signal de brouillage (S(b)) aura une phase décalée par rapport auxdits signaux d'écho ultrasonores (S(d)).

11. Procédé selon la revendication 5, le procédé comprenant en outre :
- avant ladite étape de moyennage, l'amplification dudit signal ultrasonore combiné ;
- avant ladite étape de moyennage, la conversion dudit signal ultrasonore combiné en un signal ultrasonore numérique ;
- après ladite étape de moyennage, la fourniture d'informations de synchronisation (T1) dudit dispositif générateur d'impulsions (40) à ladite unité de traitement de signaux (70), et la synchronisation desdites impulsions d'excitation ultrasonores avec ledit signal d'ablation,
dans lequel la génération d'au moins deux impulsions d'excitation ultrasonores (S(c) ; S(c1), S(c2), S(c3)) en une succession rapide est effectuée dans un mode de type rafale, chaque rafale contenant au moins quatre balayages, chaque balayage étant de préférence espacé de moins de 0,1 ms par rapport à un balayage suivant, et chaque rafale étant de préférence espacée de plus de 1 ms par rapport à une rafale suivante, ledit dispositif générateur d'impulsions (40) recevant un signal de rafale de départ (S1) afin de générer une séquence d'impulsions d'excitation, lesdites informations de synchronisation (T1) étant utilisées pour synchroniser le départ d'une rafale suivante.

12. Produit de programme informatique comprenant des moyens de code pour produire les étapes de la revendication 4 lorsqu'il est exécuté sur un dispositif informatique tel qu'utilisé dans un appareil selon la revendication 1.
